# FASCICULE DE BREVET EUROPEEN

(11) **EP 3 071 521 B1**
(45) Date de publication et mention de la délivrance du brevet: **11.05.2022**
(21) Numéro de dépôt: 14805524.7
(22) Date de dépôt: 21.11.2014
(51) Int. Cl.: G01N 33/00, G06Q 10/08, C01B 32/50

(54) **MÉTHODE DE MARQUAGE CHIMIQUE DE LOTS DE DIOXYDE DE CARBONE EN VUE D'EN ASSURER LA TRAÇABILITÉ**
VERFAHREN ZUR CHEMISCHEN MARKIERUNG VON KOHLENDIOXIDCHARGEN ZUR SICHERSTELLUNG DER VERFOLGBARKEIT
METHOD OF CHEMICAL MARKING OF BATCHES OF CARBON DIOXIDE IN ORDER TO ENSURE TRACEABILITY

(30) Priorité: 22.11.2013 FR 1302750
(43) Date de publication de la demande: 28.09.2016
(73) Titulaire: Degroote, Jacques, 75016 Paris (FR)
(72) Inventeur: Degroote, Jacques, 75016 Paris (FR)
(74) Mandataire: Cronin, Brian Harold John
(86) Numéro de dépôt international: PCT/EP2014/075320
(87) Numéro de publication internationale: WO 2015/075197

(56) Documents cités:
- EP-A1- 0 513 604
- WO-A2-03/010534
- US-A- 4 551 154
- US-B1- 7 704 746
- WELLS ET AL: "The use of tracers to assess leakage from the sequestration of CO2 in a depleted oil reservoir, New Mexico, USA", APPLIED GEOCHEMISTRY, PERGAMON, AMSTERDAM, NL, vol. 22, no. 5, 6 mai 2007 (2007-05-06), pages 996-1016, XP022063723, ISSN: 0883-2927, DOI: 10.1016/J.APGEOCHEM.2007.01.002
- GREGORY J NIMZA ET AL: "The Use of Noble Gas Isotopes for Monitoring Leakage of Geologically Stored CO2", 1 janvier 2005 (2005-01-01), GEOLOGIC STORAGE OF CARBON DIOXIDE WITH MONITORING AND VERIFICATION, ELSEVIER, AMSTERDAM, PAGE(S) 1113 - 1128, XP008107303, ISBN: 978-0-08-044572-4 [extrait le 2007-09-18] abrégé page 1113, alinéa 1 - page 1114, alinéa 1 page 1119, dernier alinéa figure 3
- MATTHEW MYERS ET AL: "Tracers - Past, present and future applications in CO2 geosequestration", APPLIED GEOCHEMISTRY, vol. 30, 1 mars 2013 (2013-03-01), pages 125-135, XP055137947, ISSN: 0883-2927, DOI: 10.1016/j.apgeochem.2012.06.001

## Description

La présente invention concerne une méthode permettant de réaliser des lots de dioxyde de carbone (CO2) marqués chimiquement en vue d'en assurer la traçabilité le long d'une chaîne logistique.

En particulier la présente invention concerne une méthode de marquage de plusieurs lots homogènes de ce gaz combinant la formulation d'un traceur chimique original pilotée par un système informatique et l'injection dudit traceur, dont la composition originale est formulée de manière à ce que le lot marqué soit unique et identifiable comme tel grâce à l'utilisation de composés traceurs utilisés en combinaison. La méthode comprend aussi l'étape de mélange desdits plusieurs lots pour former un nouveau lot de dioxyde de carbone, dont la composition du traceur, étant la combinaison pondérée par les quantités assemblées des traceurs de chaque lot en mélange, constitue une unique caractéristique pour l'identification de ce nouveau lot ainsi composé. Une étape d'analyse des traceurs permet d'identifier la composition de celui-ci. Cette méthode permet, par une comparaison de celle-ci avec la base de données du système de traçabilité, l'identification de manière certaine d'un lot, rendant possible ainsi la traçabilité tout au long de la chaine logistique.

La méthode trouve des applications dans de nombreux domaines où le dioxyde de carbone est recyclé dans des usages industriels ou agricoles, ou simplement capté et séquestré pour répondre aux enjeux climatiques.

Nous assistons à la mise en place d'une économie circulaire par laquelle le déchet d'une industrie devient la ressource de l'industrie suivante dans la chaîne de production. Ainsi les gaz de combustion des combustibles fossiles comme le pétrole, le charbon, le gaz naturel, le lignite et les hydrocarbures de roche mère, qui contiennent une grande proportion de dioxyde de carbone, sont dans la quasi-totalité des cas rejetés dans l'atmosphère, où le CO2 contribue à piéger le rayonnement thermique infrarouge et ainsi augmenter l'effet de serre et le réchauffement climatique. Or le dioxyde de carbone peut aussi être une ressource industrielle ou agricole, jusqu'à là souvent ignorée ou négligée.

Mais, à ce jour, c'est la dimension déchet qui domine la vision que les gens ont du CO2.

Le CO2 étant identifié comme un des principaux gaz à effet de serre, sa capture sur ses lieux d'émission, son utilisation industrielle, son stockage et dans certains cas sa séquestration géologique de très longue durée (plusieurs milliers d'années) sont essentiels pour éviter d'accroître davantage la contribution anthropique au réchauffement climatique.

Lorsqu'un sous-produit ou déchet d'un processus industriel est destiné au recyclage ou à la destruction, l'opérateur industriel cédant reste responsable du bon suivi de la réglementation par le cessionnaire jusqu'à l'achèvement complet du cycle de recyclage ou de destruction. Il pourrait en être de même pour les gaz de combustion et notamment le CO2. Le transfert de propriété d'un gaz identifié comme un déchet engage la responsabilité du cédant à vérifier le bon respect de la réglementation par le cessionnaire soit par la destruction du déchet, son stockage ou sa séquestration selon les réglementations du lieu, soit par son recyclage c'est-à-dire sa requalification en produit, mais dans ce cas, cette réaffectation doit répondre à des réglementations qui notamment, obligent que les éléments nécessaire à sa traçabilité originelle soient attachés au lot du gaz.

Ce cas s'applique particulièrement aux procédés de captage du CO2 industriel à des fins de réutilisation ou de séquestration environnementale qui donne lieu à des échanges fiduciaires ou des exemptions de taxes pour des sommes considérables.

Il existe plusieurs procédés et appareillages de capture du CO2 qui en font une ressource propre à être transportée vers son lieu d'utilisation ou de stockage. Ce gaz peut être transporté après compression et/ou liquéfaction en utilisant les vecteurs de transport généralement utilisé pour les gaz (navire, pipeline). Cependant, toute matière première fournie comme intrant à l'industrie, comme toute matière considérée comme un déchet, doit faire l'objet d'une traçabilité tout au long de son transport, de son lieu de captation ou d'extraction jusqu'à son lieu d'utilisation finale. Il est important que toutes les industries utilisant le CO2 comme un intrant puissent connaître la nature et l'origine de façon garantie et agréée. Chaque lot doit être caractérisé et identifiable.

À titre d'exemple, ce gaz est en termes de volume, le premier engrais au monde utilisé par la biosphère végétale grâce à la photosynthèse, s'inscrivant dans le cadre du cycle du carbone. Certaines applications émergentes comme la production de microalgues sont très consommatrices de CO2. D'autres secteurs industriels, dans le domaine de la chimie du carbone, utilisent ce gaz dans des proportions en forte croissance.

L'utilisation industrielle du CO2, au-delà de son stockage en vue de sa séquestration (CSS), peut rentrer dans de nombreux domaines applicatifs : boissons pétillantes, atmosphères inertes, extincteurs, fluide frigorigène, glace carbonique, etc. Le CO2 dans sa phase supercritique est un solvant utilisé dans les industries agroalimentaires, pharmaceutiques et cosmétiques. Le CO2 est également utilisé en agriculture sous serre, dans le but d'augmenter les rendements par sur-concentration en CO2 de l'enceinte confinée.

Dans le cas d'une séquestration géologique du CO2, celle-ci doit faire l'objet d'un agrément et d'un constat, lequel doit permettre, selon la réglementation en vigueur, l'émission de certificat pouvant avoir une valeur fiduciaire, adossable à des droits dits « droits à polluer », des quotas d'émission ou des exonérations partielles ou totales de taxes sur les activités polluantes, mais également faire l'objet d'une compensation financière dans le cadre des réglementations sur les échanges de droits carbone (que ce soit pour les pays ayant mis en place des mécanismes d'échange de droits à polluer, ou dans le cadre des Mécanismes de Développement Propre mis en place par les accords de Kyoto).

### État de l'art antérieur

L'état de la technique permet d'injecter un composé gazeux pour marquer du gaz et en permettre la détection directement par des utilisateurs ou au moyen d'une sonde reliée à un appareil de mesure. Il existe des protocoles d'ajout d'un traceur olfactif permettant la détection de fuites, comme c'est le cas pour l'usage des gaz combustibles à destination des particuliers (gaz de ville, bouteilles de butane, distribution de GPL carburant, etc.) dans lesquels des gaz soufrés, non toxiques et à très bas seuil de perception par le nez humain, sont utilisés.

Cette méthode a été généralisée dans les circuits de distribution de gaz naturel à usage domestique. En cas de fuite de ce gaz combustible, la présence du gaz adjuvant facilement reconnaissable par son odeur permet de donner l'alerte au risque d'explosion. Cette méthode permet donc d'identifier le caractère dangereux d'un gaz mais en aucun cas ne permet d'identifier le gaz (méthane, butane ou autre), de spécifier le lieu de sa synthèse ou de son captage, d'identifier le fournisseur, et encore moins de répondre à une identification par lot. Cette méthode n'est pas utilisée pour le CO2.

Il existe déjà des procédés permettant le marquage chimique de gaz :
- comme c'est le cas pour le marquage du gaz naturel stocké dans les nappes souterraines à l'aide de traceurs synthétisés par réaction catalytique du gaz tels que l'éthylène, le propylène, l'hydrogène ou le monoxyde de carbone comme présenté dans le document WO2004023095 ; dans l'invention selon ce procédé, le gaz concerné est une ressource minérale naturelle de grande valeur et l'utilisation du traceur sert à déterminer la quantité de gaz naturel dans une nappe de stockage et/ou suivre les déplacements du gaz à travers les roches ;
- comme c'est le cas en perfusant un gaz de marquage isotopique réactif dans un appareillage de spectrométrie de masse tel que décrit dans le document WO2010120895 ;
- comme le marquage de fluides, par adjonction de microparticules fluorescentes tel qu'enseigné dans le document WO9952708, créant un milieu diphasique (liquide-solide ou gaz-solide) qui peut être séparé ;

- comme l'utilisation de traceurs solides, composés d'éléments métalliques ou de leurs sels, injectés dans une formation souterraine contenant du pétrole ou du gaz naturel, décrit dans le document WO2007102023 ;
- comme des systèmes d'injection de traceurs dans les trous de forage pétroliers et gaziers qui sont ensuite détectés pour en déduire les profondeurs concernées tels que décrits dans les documents WO9806930 et WO9533121 ;
- comme l'utilisation des gaz rares (document WO03010534) ou d'un isotope radioactif du carbone (document WO2010132295) pour détecter les mouvements souterrains et les fuites dans des installations de séquestration géologique de CO2 ;
- comme l'ajout d'un gaz traceur artificiel (SF6 ou C2HF5) au méthane stocké dans un réservoir souterrain tari afin d'en prouver la propriété, ainsi que l'enseigne le document US4551154.

Il est possible de produire par réaction chimique des mélanges de gaz à plusieurs composantes au moment de l'injection dans un flux de gaz tel que le processus décrit dans le document WO0125781, mais cette méthode ne permet pas de pouvoir certifier la composition exacte des produits de la réaction.

Par ailleurs, le marquage des lots de CO2 en vue de leur traçabilité lors d'une séquestration souterraine est connu du document US7704746.

Il est également décrit dans les publications suivantes : A.W. Wells et al: The use of tracers to assess leakage from the sequestration of CO2 in a depleted oil reservoir, Applied Geochemistry 22 (2007), 996-1016 ; G.J. Nimza et al.: The Use of Noble Gas Isotopes for Monitoring Leakage of Geologically Stored CO2, Carbon Dioxide Capture for Storage in Deep Geologic Formations, Elsevier, Amsterdam 2005, pages 1113-1128 ; et M. Myers et al.: Tracers - Past, present and future applications in CO2 geosequestration, Applied Geochemistry 30 (2013), 125-135.

Ces méthodes permettent de suivre le cheminement d'un gaz dans un circuit ou dans un stockage souterrain, elles permettent aussi de distinguer un gaz d'origine industrielle d'un gaz d'origine naturelle. Mais il est impossible de pouvoir créer à volonté des lots de gaz différents comportant des marqueurs originaux et stables, aptes à être couplés à un système de traçabilité sécurisé.

Par ailleurs, il existe des procédés de détection de traceurs de gaz :
- la détection directe par l'homme est possible pour des traceurs ayant une forte empreinte olfactive, tels les mercaptans utilisés comme traceurs olfactifs à des fins d'alerte dans les réseaux de gaz naturel ;
- une détection indirecte via une réaction chimique colorée impliquant ces mêmes mercaptans comme évoqué dans le document WO0002029 ;
- la détection de traceurs fluorescents pour repérer les fuites dans les circuits de climatisation est bien connue de l'homme du métier ; la fluorescence est aussi utilisée pour marquer des réservoirs d'hydrocarbures selon la demande US20100208260 ;
- la détection de gaz traceurs à très faible concentration via un capteur chimique spécifique tel qu'utilisé dans le document DE102011007332. En outre, le document EP0513604 divulgue un système d'identification de fluides hydrocarbonés basé sur l'injection d'une permutation unique d'une pluralité prédéterminée de composants traceurs.

Il est possible plus généralement de repérer les contenants de gaz, en particulier les gaz sous pression, par des systèmes mécaniques de marquage individuel et d'identification courants sur les bouteilles ou citernes de gaz ; le document WO2011104736 en présente un cas particulier. Néanmoins, la notion d'identification de lot de gaz homogène ne se fait alors que par l'identification du contenant, mais ce n'est pas le gaz en lui-même qui fait l'objet d'une sérialisation originale, lot par lot. Ainsi, en cas de transfert du gaz d'un contenant à un autre, ou de passage par une installation de liquéfaction / gazéification, la chaîne de traçabilité est rompue ; rien n'interdit non plus de vider puis remplir à nouveau le contenant avec un autre lot de gaz.

La méthode de production de lot de CO2 marqué consiste à injecter dans des lots de gaz issus d'un processus industriel produisant du CO2 (par exemple une combustion, une fermentation), directement ou après traitement (lavage, enrichissement, séparation), un mélange de composés chimiques marqueurs dont la composition est créée spécifiquement pour le lot à marquer, afin d'en permettre l'identification physique ou chimique tout au long de sa chaîne logistique, même en cas de changement d'état, de transbordement, de changement de contenant, etc. Le transport de ce lot de gaz peut se faire dans des enceintes confinées, par voie terrestre, fluviale, maritime ou par pipeline.

Le mode de réalisation envisage également - l'assemblage de plusieurs lots par mélange constituant ainsi un nouveau lot homogène, dont la composition du traceur, étant alors elle-même la combinaison, pondérée par les quantités assemblées, des traceurs de chaque lot en mélange, sera unique et caractéristique de ce nouveau lot ainsi composé.

Le lot homogène de CO2 marqué de façon unique est composé :
- Pour une part très majoritaire (au moins 90% en masse), de dioxyde de carbone, par exemple issu de la combustion de ressources fossiles telles que le charbon, le pétrole et le gaz naturel
- Pour une part minoritaire (moins de 5% en masse), d'un traceur sous la forme d'une combinaison de plusieurs (au minimum deux) composés chimiques.

La méthode de marquage d'un lot de dioxyde de carbone, comprend les étapes de:
- Création par un système informatique de traçabilité d'une référence pour un lot de dioxyde de carbone par un système informatique
- Injection d'un traceur chimique dans le lot de dioxyde de carbone, la concentration finale du traceur dans le lot étant inférieure à 1% en masse
- Enregistrement de la formule du traceur chimique dans le système informatique de traçabilité
caractérisée par le fait que la formule du traceur chimique est spécifique du lot et se présente sous la forme d'une combinaison d'au moins deux substances chimiques.

Selon un mode avantageux de réalisation, la concentration des substances du traceur chimique dans le lot marqué de CO2 est inférieure à 0,1% (1000 ppm).

Pour la réalisation du traceur, on peut avantageusement utiliser des composés chimiques choisis parmi :
- Des composés ou des hydrocarbures halogénés, tels que, mais non limitativement, l'hexafluorure de soufre, les hydroflurorocarbones, les hydrochlorofluorocarbones, les chlorofluorocarbones et les hydrochlorocarbones
- Des composés organiques oxygénés tels que ceux appartenant à la famille des alcools, des aldéhydes, des cétones, des éthers, des acides carboxyliques
- Des composés organiques soufrés tels que les thiols ou des composés azotés

Pour des raisons économiques, les composés traceurs seront préférentiellement choisis parmi les composés combinant un seuil de détection très faible (typiquement de l'ordre du ppm, voire beaucoup plus faible) et un prix de revient modéré.

Avantageusement, les composés peuvent être des énantiomères ou diastéréoisomères présents dans des proportions non racémiques ; leurs propriétés physiques et chimiques étant très proches, le ratio entre composés évolue peu lors des transformations subies le long de la chaîne logistique du lot de CO2.

Avantageusement, deux au moins des composés peuvent être des énantiomères ou diastéréoisomères ou diffèrent seulement par leur composition isotopique.

On peut également, pour mettre en œuvre la méthode, utiliser des composés chimiques enrichis en isotopes naturels stables comme le deutérium ou le carbone 13, uniformément ou à certaines positions bien précises des molécules.

On peut également, pour mettre en œuvre la méthode, utiliser des composés chimiques fluorescents.

L'objet de l'invention intègre l'utilisation d'un système informatique, de préférence fortement sécurisé, destiné à :
- référencer chaque lot, avec des informations comme le propriétaire du gaz, la période de captation du gaz constituant le lot, la volumétrie de gaz, l'installation industrielle à l'origine du lot etc.
- concevoir une formule chimique de traceur pour ce lot
- associer cette formule au lot
- piloter à distance sa réalisation et son injection dans le lot, via le contrôle d'un automate implanté dans l'installation industrielle et équipé de réservoirs de substances chimiques et d'électrovannes
- le cas échéant, gérer l'assemblage de plusieurs lots afin de constituer un lot plus important, le traceur de ce lot assemblé étant la combinaison linéaire des traceurs de chaque lot individuel
- enregistrer chaque événement de la chaîne logistique du lot marqué de CO2 ainsi constitué
- analyser les résultats des analyses physico-chimiques réalisées aux fins d'identification d'un lot de CO2 et comparer avec la composition théorique du traceur et conclure grâce à un système expert (tenant compte des différentes transformations subies par le lot et ayant pu affecter la composition du traceur) sur l'identification du lot.

Selon un mode de réalisation, l'injection est réalisée dans le gaz liquéfié et comprimé.

Selon un autre mode de réalisation, l'injection est réalisée sous forme gazeuse.

Le système informatique peut prendre en compte les aléas de fonctionnement, notamment au moment de l'injection (dysfonctionnement d'une vanne, bouteille vide, etc.) en recalculant automatiquement une nouvelle formule de traceur.

Le système informatique est certifié et auditable par des organismes de contrôle agréés de manière à pouvoir faire foi dans le cadre de systèmes de régulation et de compensation liés au CO2 (taxes sur les activités polluantes, taxes carbone, quotas d'émission, mécanismes de développement propre, etc.).

Il est obtenu un lot marqué de dioxyde de carbone, contenant un traceur chimique et référencé dans un système informatique de traçabilité, caractérisé par le fait que :
- Le lot contient un minimum de 90% en masse de dioxyde de carbone
- Le traceur chimique est composé d'un mélange d'au moins deux substances chimiques, la concentration du traceur dans le lot étant inférieure à 1% en masse
- Le système informatique de traçabilité associe dans une base de données le lot marqué à la proportion des différents composants du traceur et à des informations sur l'origine du dioxyde de carbone
- Le lot peut être analysé afin d'identifier la nature et la proportion des substances composant le traceur chimique.

En particulier, dans le lot décrit ci-avant, l'une au moins des substances chimiques du traceur est un hydrocarbure halogéné, un composé soufré, azoté ou oxygéné.

Alternativement, dans le lot décrit ci-avant, au moins deux des substances chimiques du traceur sont des énantiomères ou des diastéréoisomères ou diffèrent seulement par leur composition isotopique.

Les analyses propres à la détection des substances chimiques composant le mélange et à l'identification du lot sont réalisées de façon préférentielle par spectrométrie de masse ou chromatographie en phase gazeuse ou par résonance magnétique nucléaire. D'autres techniques analytiques peuvent être employées comme la spectroscopie (visible, UV, IR ou FT-IR). Alternativement, la chromatographie, l'électrophorèse, peuvent être utilisées, notamment avec une colonne comportant une phase chirale permettant ainsi de séparer des molécules énantiomères ou diastéréoisomères.

Alternativement, la méthode d'analyse physico-chimique est réalisée par une combinaison de ces méthodes analytiques.

### Avantages apportés par l'invention

La méthode présente plusieurs avantages par rapport à l'art antérieur, particulièrement en ce qu'elle permet de réaliser autant de lots différents que nécessaire grâce aux combinaisons en proportions variables de différents composés chimiques utilisés comme traceurs.

Ce système peut s'inscrire dans un dispositif de traçabilité complet de gaz et permet notamment l'assemblage de lots différents lors d'un changement de mode de transport ou de vecteurs, et d'une rupture de charge. La connaissance de la composition de chacun des lots marqués permet d'en déduire la composition résultante après mélange et de l'identifier de façon aussi fiable que chacun des lots originaux.

Ce système permettant la traçabilité des lots de CO2 apporte une réponse à des cahiers des charges industriels sur la nature du gaz, le volume du lot, la destination du lot, la localisation du lot, l'origine du lot, quand bien même le lieu de production du lot et le lieu d'utilisation sont éloignés de plusieurs milliers de kilomètres.

Le système peut avantageusement être utilisé par les industriels souhaitant prouver que le CO2 produit par leur installation a bien été réutilisé, recyclé ou enfoui.

Le système peut aussi servir à un industriel utilisateur de CO2 et désireux de certifier de certifier que le gaz utilisé a une caractéristique donnée : généré par un processus propre ou biologique (par ex. une fermentation), produit sur un territoire donné, etc. Cette fonctionnalité peut intéresser des industriels de l'agroalimentaire désireux de maîtriser la qualité de leurs intrants y compris gazeux (production biologique, cultures de microalgues, etc.) ; pour une telle application, les composés traceurs seront choisis par les composés naturels autorisés par les normes alimentaires.

## Revendications

1. Méthode de marquage d'un lot de dioxyde de carbone formé d'un mélangede plusieurs lots, chaque lot comprenant du dioxyde de carbone recyclé dans des usages industriels ou agricoles ou capté et séquestré pour répondre aux enjeux climatiques, la méthode comprenant pour chaque lot les étapes a. b. c suivantes :
a. Création par un système informatique de traçabilité d'une référence du lot de dioxyde de carbone dans une base de données d'un système informatique répertoriant une pluralité de lots de dioxyde de carbone;
b. Injection d'un traceur chimique dans le lot de dioxyde de carbone, la concentration finale du traceur dans chaque lot étant inférieure à 1% en masse ;
c.Enregistrement de la formule du traceur chimique du lot dans le système informatique de traçabilité; où la formule du traceur chimique est spécifique du lot et se présente sous la forme d'une combinaison d'au moins deux substances chimiques;
Ensuite :
Mélange desdits plusieurs lots pour former un nouveau lot de dioxyde de carbone, dont la composition du traceur, étant la combinaison pondérée par les quantités assemblées des traceurs de chaque lot en mélange, constitue une unique caractéristique pour l'identification de ce nouveau lot ainsi composé.

2. Méthode selon la revendication 1, où l'une au moins des substances chimiques est :
a. un hydrocarbure halogéné ; ou
b. un composé soufré ou azoté.

3. Méthode selon la revendication 1, où l'une au moins des substances possède une fonction alcool, éther, acide carboxylique, aldéhyde ou cétone.

4. Méthode selon la revendication 1 où deux au moins des substances du traceur sont des énantiomères ou des diastéréoisomères ou diffèrent seulement par leur composition isotopique.

5. Méthode selon la revendication 1 où l'injection est réalisée dans le gaz liquéfié et comprimé.

6. Méthode selon la revendication 1 où l'injection est réalisée sous forme gazeuse.

7. Méthode selon l'une des revendications 1 à 6 où la concentration des substances du traceur chimique injectées dans chaque lot de CO2 est inférieure à 0,1% (1000 ppm) en masse.

## Patentansprüche

1. Verfahren zur Kennzeichnung einer Kohlendioxidcharge, die aus einer Mischung mehrerer Chargen besteht, wobei jede Charge Kohlendioxid enthält, das für industrielle oder landwirtschaftliche Zwecke recycelt oder aufgefangen und sequestriertwurde, um den Klimaherausforderungen zu begegnen, wobei das Verfahren für jede Charge die folgenden Schritte a. b. c. umfasst:
a. Erstellung einer Referenz der Kohlendioxid-Charge durch ein Computer-Rückverfolgungssystem in einer Datenbank eines Computersystems, das eine Vielzahl von Kohlendioxid-Chargen auflistet;
b. Einspritzen eines chemischen Tracers in die Kohlendioxidcharge, wobei die Endkonzentration des Tracers in jeder Charge weniger als 1 Massenprozent beträgt;
c. Aufzeichnung der Formel des chemischen Tracers der Charge im computergestützten System zur Rückverfolgbarkeit;
Wobei die Formel des chemischen Tracers chargenspezifisch ist und als Kombination von mindestens zwei chemischen Substanzen vorliegt;
Anschließend :
Mischen der genannten Chargen, um eine neue Charge Kohlendioxid zu bilden, wobei die Zusammensetzung des Tracers als gewichtete Kombination der zusammengesetzten Mengen der Tracer in jeder gemischten Charge ein einziges Merkmal zur Identifizierung dieser neuen, so zusammengesetzten Charge darstellt.

2. Verfahren nach Anspruch 1, wobei mindestens eine der Chemikalien ist :
a. ein halogenierter Kohlenwasserstoff; oder
b. eine Schwefel- oder Stickstoffverbindung.

3. Verfahren nach Anspruch 1, wobei mindestens eine der Substanzen eine Alkohol-, Ether-, Carbonsäure-, Aldehyd- oder Ketonfunktion besitzt.

4. Verfahren nach Anspruch 1, bei dem mindestens zwei der Tracersubstanzen Enantiomere oder Diastereoisomere sind oder sich nur in ihrer Isotopenzusammensetzung unterscheiden.

5. Verfahren nach Anspruch 1, bei dem die Injektion in verflüssigtes und komprimiertes Gas erfolgt.

6. Verfahren nach Anspruch 1, bei dem die Injektion in Gasform durchgeführt wird.

7. Verfahren nach einem der Ansprüche 1 bis 6, bei dem die Konzentration der injizierten chemischen Tracer-Substanzen in jeder CO2-Charge weniger als 0,1 Massenprozent (1000 ppm) beträgt.

## Claims

1. A method of marking a batch of carbon dioxide formed from a mixture of several batches, each batch comprising carbon dioxide recycled in industrial or agricultural uses or captured and sequestered to address climate issues, the method comprising for each batch the following steps a. b. c:
a. Creation by a traceability computer system of a reference to the batch of carbon dioxide in a database of a computer system listing a plurality of batches of carbon dioxide;
b. Injection of a chemical tracer into the batch of carbon dioxide, the final concentration of tracer in each batch being less than 1% by mass;
c. Recording the chemical tracer formula of the batch in the computerised traceability system;
Where the chemical tracer formula is batch specific and is in the form of a combination of at least two chemicals;
Then :
Blending of the said several batches to form a new batch of carbon dioxide, the tracer composition of which, being the combination weighted by the assembled amounts of the tracers of each batch in the blend, constitutes a single characteristic for the identification of this new batch so composed.

2. The method of claim 1, wherein at least one of the chemicals is :
a. a halogenated hydrocarbon; or
b. a sulphur or nitrogen compound.

3. The method of claim 1, wherein at least one of the substances has an alcohol, ether, carboxylic acid, aldehyde or ketone function.

4. The method of claim 1 wherein at least two of the tracer substances are enantiomers or diastereomers or differ only in their isotopic composition.

5. The method according to claim 1 wherein the injection is carried out into the liquefied and compressed gas.

6. The method of claim 1 wherein the injection is carried out in gaseous form.

7. A method according to any of claims 1 to 6 wherein the concentration of the chemical tracer substances injected into each batch of CO2 is less than 0.1% (1000 ppm) by mass.
